# EUROPÄISCHE PATENTANMELDUNG

(11) **EP 1 143 003 A2**
(43) Veröffentlichungstag der Anmeldung: **10.10.2001**
(21) Anmeldenummer: 01105928.4
(22) Anmeldetag: 09.03.2001
(51) Int. Cl.: C12N 15/31, C07K 14/34, C12N 1/21, C12P 13/08, C12Q 1/68

(54) **Nukleotidsequenzen, die für das rplk-Gen kodieren, und Verfahren zur Herstellung von L-Aminosäuren**

(30) Priorität: 05.04.2000 DE 10017057; 10.05.2000 US 568023
(71) Anmelder: Degussa AG, 40474 Düsseldorf (DE)
(72) Erfinder: Wehmeier, Lutz, Dr., 30900 Wedemark-Meitze (DE); Tauch, Andreas, Dr., 33647 Bielefeld (DE); Pühler, Alfred, Prof., 33739 Bielefeld (DE); Kalinowski, Jörn, Dr., 33615 Bielefeld (DE); Möckel, Bettina, Dr., 40597 Düsseldorf (DE)

(57) **Zusammenfassung**

Isoliertes Polynukleotid aus coryneformen Bakteren, enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c),
und Verfahren zur fermentativen Herstellung von L-Aminosäuren unter Abschwächung des rplK-Gens.

## Beschreibung

Gegenstand der Erfindung sind für das rplK-Gen kodierende Nukleotidsequenzen aus coryneformen Bakterien und ein Verfahren zur fermentativen Herstellung von Aminosäuren, insbesondere L-Lysin, unter Abschwächung des rplK-Gens.

### Stand der Technik

L-Aminosäuren, insbesondere L-Lysin, finden in der Tierernährung, in der Humanmedizin und in der pharmazeutischen Industrie Anwendung.

Es ist bekannt, daß diese Aminosäuren durch Fermentation von Stämmen coryneformer Bakterien, insbesondere Corynebacterium glutamicum, hergestellt werden. Wegen der großen Bedeutung wird ständig an der Verbesserung der Herstellungsverfahren gearbeitet. Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung L-Aminosäure produzierender Stämme von Corynebacterium eingesetzt, indem man einzelne Aminosäure-Biosynthesegene amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

Das RplK-Protein (ribosomal large subunit protein K) ist ein zuerst für Escherichia coli beschriebener Bestandteil des bakteriellen Ribosoms, dem Translationsapparat der Zelle, an dem die Proteinsynthese erfolgt.

Ribosomen sind zelluläre Partikel, die sich aus drei Ribonukleinsäure(RNA)-Molekülen und einer definierten Anzahl an Proteinen zusammensetzen. Ribosomen werden meist aus Zellextrakten durch Ultrazentrifugation gewonnen. Die weitere Aufreinigung von den übrigen Zellbestandteilen erfolgt üblicherweise durch Sedimentation im Saccharose-Gradienten. Diese Technik der Präparation hat zu den gebräuchlichen Bezeichnungen für die Bestandteile der Ribosomen geführt, die direkt Bezug auf die Sedimentationseigenschaften nehmen. Ein funktionelles bakterielles Ribosom wird aus diesem Grund oft als 70S-Ribosom bezeichnet, das aus der kleinen (small subunit) 30S-Untereinheit und der großen (large subunit) 50S-Untereinheit besteht. Die kleine 30S-Untereinheit von E. coli besteht aus 21 verschiedenen Polypeptiden und einem RNA-Molekül mit einer Länge von 1542 Nukleotiden, dem Fachmann als 16S-rRNA bekannt; die große 50S-Untereinheit enthält neben dem RplK-Protein weitere 31 unterschiedliche Polypeptide, zusammen mit zwei RNA-Molekülen mit einer Länge von 120 bzw. 2904 Nukleotiden, den sogenannten 5S-bzw. 23S-rRNA-Molekülen. Inzwischen hat sich für die ribosomalen Proteine eine alternative Nomenklatur etabliert. So werden die Polypeptide der kleinen 30S-Untereinheit vom Fachmann mit S1 bis S21, die der großen 50S-Untereinheit mit L1 bis L32 bezeichnet. Das RplK-Protein entspricht dabei dem ribosomalen L11-Protein (Noller and Nomura, In: Neidhardt et al. Escherichia coli and Salmonella typhimurium: Cellular and molecular biology. American Society for Microbiology, Washington D.C., 167-186, 1996). In den letzten Jahren wurden L11-ähnliche Proteine auch in anderen Organismen wie Borrelia burgdorferi (Fraser et al., Nature, 390, 580-586, 1997), Helicobacter pylori (Tomb et al., Nature, 388, 539-547, 1997), Serratia marcescens (Sor and Nomura, Molecular and general Genetics, 210, 52-59, 1987), Haemophilus infuenzae (Fleischmann et al., Science, 269, 496-512, 1995) und in dem gram positiven Bakterium Bacillus subtilis (Jeong et al., Molecular Microbiology, 10, 133-142, 1993) identifiziert.

Der am Ribosom ablaufende Prozeß der Translation, also die Matrizen-RNA(mRNA)-gelenkte Biosynthese von Polypeptiden, ist komplex. Neben dem Ribosom sind weitere Proteine für den Translationsprozeß essentiell, die vom Fachmann als Proteinsynthese-Faktoren bezeichnet werden (Noller, Annual Review of Biochemistry, 60, 191-227, 1991). Das L11-Protein vermittelt die Interaktion zwischen dem Ribosom und einigen Proteinsynthese-Faktoren; als Beispiel können hier der Elongations-Faktor G (EF-G) und der Terminations-Faktor 1 (RF-1) genannt werden. Das Fehlen des L11-Proteins in den Ribosomen von E. coli L11-Mutanten führt daher zu einer Verringerung der Translationsrate (Xing and Draper, Biochemistry, 35, 1581-1588, 1996).

Das L11-Protein ist ebenfalls essentiell für die Bindung und Aktivität des RelA-Proteins am Ribosom. RelA katalysiert unter Aminosäure-Mangelbedingungen die Synthese von Guanosintetraphosphat (ppGpp) durch die Übertragung einer Pyrophosphatgruppe von ATP auf GDP. ppGpp beeinflußt in E. coli die Expression vieler Gene, entweder negativ oder positiv. Im allgemeinen wird dabei die Expression von Genprodukten, die in biosynthetischen Wegen wirksam sind, stimuliert. Genprodukte, die katabolisch wirksam sind, werden in der Regel entsprechend negativ reguliert. Durch ppGpp werden in E. coli eine große Zahl von Genen und Operons reguliert, die in der Aminosäurebiosynthese eine zentrale Rolle spielen. Zu den bisher bekannten in E. coli positiv beeinflußten Genen gehören u. a. argF, argI, argECBH (Arginin-Biosynthese), gltB, glnA, gdh (Glutamin/Glutamat-Biosynthese), ilvB, IlvA (Isoleucin-Biosynthese), metC, metF, metK (Methionin-Biosynthese), thrA, thrB, thrC (Threonin-Biosynthese), lysA, lysC, dapB, asd (Lysin-Biosynthese) (Cashel et al., In: Neidhardt et al. Escherichia coli and Salmonella typhimurium: Cellular and molecular biology. American Society for Microbiology, Washington D.C., 1458-1496, 1996). Die Funktion von ppGpp als positiver Regulator der Aminosäure-Biosynthese wurde inzwischen auch in anderen Bakterien wie Salmonella typhimurium (Rudd et al., Journal of Bacteriology, 163, 534-542, 1985), Vibrio sp. (Flärdh et al., Journal of Bacteriology, 176, 5949-5957, 1994) und B. subtilis (Wendrich and Marahiel, Molecular Microbiology, 26, 65-79, 1997) gezeigt.

Aus dem Stand der Technik wird deutlich, daß ein Interesse daran besteht, herauszufinden, ob die Kenntnis der Nukleotidsequenz des rplK-Gens coryneformer Bakterien zur Verbesserung der Aminosäureproduktion dieser Bakterien beirägt.

### Aufgabe der Erfindung

Die Erfinder haben sich zur Aufgabe gestellt neue Maßnahmen zur verbesserten fermentativen Herstellung von Aminosäuren, insbesondere L-Lysin, zur Verfügung zu stellen.

### Beschreibung der Erfindung

L-Aminosäuren, insbesondere Lysin, finden in der Humanmedizin und in der pharmazeutischen Industrie, in der Lebensmittelindustrie und ganz besonders in der Tierernährung Anwendung. Es besteht daher ein allgemeines Interesse daran, neue Ansätze für verbesserte Verfahren zur Herstellung von Aminosäuren, insbesondere L-Lysin, bereitzustellen.

Gegenstand der Erfindung ist ein isoliertes Polynukleotid enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

### Weitere Gegenstände sind

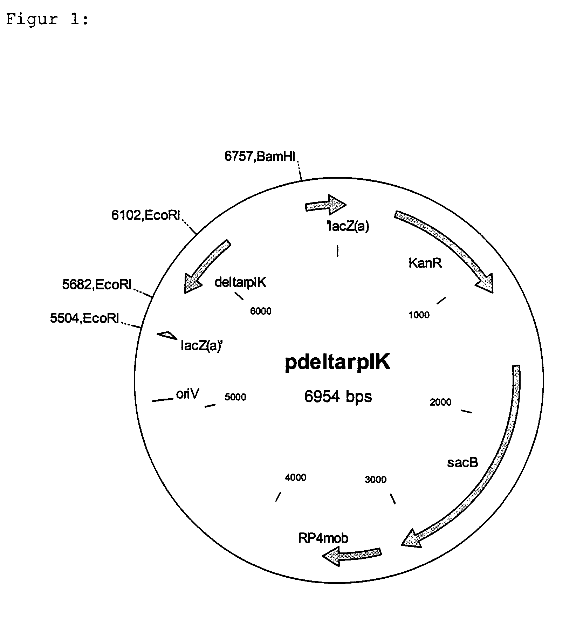

ein Polynukleotid gemäß Anspruch 4, enthaltend die Nukleotidsequenz wie in SEQ ID No. 1 dargestellt,
ein Polynukleotid gemäß Anspruch 2, das für ein Polypeptid kodiert, das die Aminosäuresequenz, wie in SEQ ID No. 2 dargestellt, enthält,
ein Polynukleotid gemäß Anspruch 1, insbesondere Punkt d, enthaltend die Nukleotidsequenz, wie in SEQ ID No. 3 dargestellt,
ein Polynukleotid gemäß Anspruch 1, Punkt d, das für ein Polypeptid kodiert, das die Aminosäuresequenz, wie in SEQ ID No. 4 dargestellt, enthält,
ein Vektor, enthaltend ein mutiertes Polynukleotid gemäß Anspruch 1, Punkt d dargestellt in SEQ ID No. 3 und Figur 1 (Δ = deHa) und nach dem Budapester Vertrag hinterlegt in E.coli DH5α/pΔrplK als DSM 13158,
und als Wirtszelle dienende coryneforme Bakterien die in dem rplk-Gen eine Insertion oder Deletion enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für das ribosomale Protein L11 kodieren und solche cDNA oder Gene zu isolieren, die eine hohe Ähnlichkeit der Sequenz mit der des rplK-Gens aufweisen.

Polynukleotidsequenzen gemäß der Erfindung sind weiterhin als Primer geeignet, mit deren Hilfe mit der Polymerase Kettenreaktion (PCR) DNA von Genen hergestellt werden kann, die für das RplK-Genprodukt bzw. ribosomale Protein L11 kodieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Basenpaaren.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen das Polypeptid gemäß SEQ ID No. 2, insbesondere solche mit der biologischen Aktivität des RplK-Genproduktes und auch solche ein, die zu wenigstens 70% identisch sind mit dem Polypeptid gemäß SEQ ID No. 2, bevorzugt zu wenigstens 80% und besonders zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren, insbesondere Lysin, unter Verwendung von coryneformen Bakterien, die insbesondere bereits die Aminosäuren produzieren und in denen die für das rplK-Gen kodierenden Nukleotidsequenzen abgeschwächt sind.

Der Begriff "Abschwächung" beschreibt in diesem Zusammenhang die Verringerung oder Ausschaltung der intrazellulären Aktivität bzw. Funktion eines oder mehrerer Enzyme bzw. Proteine in einem Mikroorganismus, die durch die entsprechende DNA kodiert werden, indem man beispielsweise einen schwachen Promotor verwendet oder ein Gen bzw. Allel verwendet, das für ein entsprechendes Enzym bzw. Protein mit einer niedrigen Aktivität kodiert oder das entsprechende Gen oder Enzym bzw. Protein inaktiviert und gegebenenfalls diese Maßnahmen kombiniert.

Die Mikroorganismen, die Gegenstand der vorliegenden Erfindung sind, können Aminosäuren, insbesondere Lysin, aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme,
wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DSM 5715
Corynebacterium glutamicum DSM 12866 und
Corynebacterium glutamicum DM58-1

Den Erfindern gelang es das neue rplK-Gen von Corynebacterium glutamicum zu isolieren.

Zur Isolierung des rplK-Gens von C. glutamicum wird zunächst eine Genbank von Corynebacterium glutamicum angelegt. Das Anlegen von Genbanken ist in allgemein bekannten Lehrbüchern und Handbüchern beschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)).

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde.

Die mit Hilfe von Cosmiden klonierten langen DNA-Fragmente können anschließend wiederum in gängige, für die Sequenzierung geeignete Vektoren subkloniert werden.

Methoden zur DNA-Sequenzierung sind unter anderem bei Sanger et al. (Proceedings of the National of Sciences of the United States of America USA, 74:5463-5467, 1977) beschrieben.

Auf diese Weise wurde die neue für das rplK-Gen kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des RplK-Genproduktes bzw. des L-11 Proteins dargestellt.

Kodierende DNA-Sequenzen, die sich aus SEQ ID NO 1 durch die Degeneriertheit des genetischen Kodes ergeben, sind ebenfalls Bestandteil der Erfindung. In gleicher Weise sind DNA-Sequenzen, die mit SEQ ID NO 1 oder Teilen von SEQ ID NO 1 hybridisieren, Bestandteil der Erfindung. Aminosäuresequenzen, die sich in entsprechender Weise aus SEQ ID NO 2 ergeben sind ebenfalls Bestandteil der Erfindung.

Die Erfinder fanden heraus, daß coryneforme Bakterien nach Abschwächung des rplK-Gens in verbesserter Weise L-Aminosäuren, insbesondere L-Lysin, produzieren.

Zur Erzielung einer Abschwächung können entweder die Expression des rplK-Gens oder vorzugsweise die funktionellen Eigenschaften des Proteins herabgesetzt werden. Gegebenenfalls können beide Maßnahmen kombiniert werden.

Die Verringerung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Patek et al. (Microbiology 142: 1297 (1996) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der funktionellen Eigenschaften von Proteinen führen, sind aus dem Stand der Technik bekannt. Als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs, Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Aktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations) in deren Folge falsche Aminosäuren eingebaut werden oder die Translation vorzeitig abbricht. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Methoden zur Herstellung von Mutationen mit Hilfe der Polymerase-Kettenreaktion (PCR) sind bei Newton C.R., Graham A. "PCR" 2. Auflage, Spektrum Akademischer Verlag, Heidelberg, 1997, beschrieben.

Ein Beispiel für ein Plasmid mit dessen Hilfe eine Deletionsmutagenese des rplK-Gen durchgeführt werden kann, ist das in Figur 1 dargestellte Plasmid pΔrplK.

Plasmid pΔrplK besteht aus dem von Jäger et al. (Journal of Bacteriology, 1992, 174: 5462-65) beschriebenen Plasmid pK18mobsacB, in das ein Allel des rplK-Gens, dargestellt in SEQ ID No. 3 eingebaut wurde. Das als ΔrplK bezeichnete Allel trägt eine 12 bp lange Deletion im im 5'-Bereich des Gens. Die von dem ΔrplK-Allel kodierte Variante des Proteins L11 ist als SEQ ID No. 4 dargestellt. Die dargestellte Variante des Proteins L 11 unterscheidet sich von der Wildtypform des Proteins L 11 durch das Fehlen des Tetrapeptides "Prolin-Alanin-Leucin-Glycin" entsprechend den Aminosäuren der Position 30 bis 33 von SEQ ID No. 2.

Das Plasmid pΔrplK führt nach homologer Rekombination zum Austausch des chromosomalen rplK-Gens gegen das ΔrplK-Allel. Anleitungen und Erläuterungen zur Insertionsmutagenese findet man beispielsweise bei Schwarzer und Pühler (Bio/Technology 9,84-87 (1991)) oder Fitzpatrick et al. (Applied Microbiology and Biotechnology 42, 575-580 (1994)).

Zusätzlich kann es für die Produktion von L-Aminosäuren, insbesondere L-Lysin, vorteilhaft sein, zusätzlich zur Abschwächung des rplK-Gens eines oder mehrere Enzyme des jeweiligen Biosyntheseweges zu verstärken, insbesondere zu überexprimieren.

So kann beispielsweise, insbesondere für die Herstellung von L-Lysin, eines oder mehrere der Gene, ausgewählt aus der Gruppe
- das für die Dihydrodipicolinat-Synthase kodierende dapA-Gen (EP-B 0 197 335),
- ein für eine feed back resistente Aspartatkinase kodierendes lysC-Gen (Kalinowski et al. (1990), Molecular and General Genetics 224: 317-324),
- das für die Pyruvat Carboxylase kodierende pyc-Gen (Eikmanns (1992), Journal of Bacteriology 174:6076-6086),
- das für die Malat-Chinon-Oxidoreduktase kodierende mqo-Gen (Molenaar et al., European Journal of Biochemistry 254, 395 - 403 (1998)),
- das für den Lysin-Export kodierende lysE-Gen (DE-A-195 48 222),
- das zwa1-Gen (DE 199 59 328.0; DSM 13115),
gleichgzeitig verstärkt, insbesondere überexprimiert werden.

Weiterhin kann es für die Produktion von L-Aminosäuren vorteilhaft sein, zusätzlich zu der Abschwächung des rplK-Gens gleichzeitig eines oder mehrere der Gene, ausgewählt aus der Gruppe:
- das für die Phosphoenolpyruvat-Carboxykinase kodierende pck-Gen (DE 199 50 409.1; DSM 13047),
- das für die Glucose-6-Phosphat-Isomerase kodierende pgi-Gen (US 09/396,478, DSM 12969),
- das für die Pyruvat-Oxidase kodierende poxB-Gen (DE 199 51 975.7; DSM 13114),
- das zwa2-Gen (DE: 199 59 327.2; DSM 13113),
- das für die PPGPP-Synthetase I kodierende relA-Gen (EC 2.7.6.5)
abzuschwächen.

Weiterhin kann es für die Produktion von L-Aminosäuren vorteilhaft sein, neben der Überexpression der 6-Phosphogluconat-Dehydrogenase unerwünschte Nebenreaktionen auszuschalten (Nakayama: "Breeding of Amino Acid Producing Micro-organisms" , in: Overproduction of Microbial Products, Krumphanzl, Sikyta, Vanek (eds.), Academic Press, London, UK, 1982).

Die das mutierte Polynukleotid gemäß Anspruch 1 Punkt d enthaltenden Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren insbesondere L-Lysin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten werden Sauerstoff oder Sauerstoff haltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Folgender Mikroorganismus wurde bei der Deutschen Sammlung für Mikrorganismen und Zellkulturen (DSMZ, Braunschweig, Deutschland) gemäß Budapester Vertrag hinterlegt:
- Escherichia coli Stamm DH5α/pΔrplK als DSM 13158

### Beispiele

Die vorliegende Erfindung wird im folgenden anhand von Ausführungsbeispielen näher erläutert.

### Beispiel 1

Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) mit 100 mg/l Ampicillin ausplattiert wurden. Nach Inkubation über Nacht bei 37°C wurden rekombinante Einzelklone selektioniert.

### Beispiel 2

### Isolierung und Sequenzierung des rplK-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l/ Zeocin ausplattiert. Die Plasmidpräparation der rekombinanten Klone erfolgte mit dem Biorobot 9600 (Product No. 900200, Qiagen, Hilden, Germany). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Germany) eingesetzt. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Germany).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID NO 1. dargestellt. Die Analyse der Nukleotidsequenz ergab ein offenes Leseraster von 438 Basenpaaren, welches als rplK-Gen bezeichnet wurde. Das rplK-Gen kodiert für ein Polypeptid von 145 Aminosäuren dargestellt in SEQ ID No. 2.

### Beispiel 3

### Herstellung eines Vektors mit einer Kopie des rplK-Gens

Ein chromosomales 1200 bp DNA-Fragment, welches das rplK-Gen von C. glutamicum beinhaltet, wurde mittels PCR kloniert.

Hierzu wurde chromosomale DNA aus Corynebacterium glutamicum ATCC 13032 wie bei Tauch et al. (1995, Plasmid 33:168-179)beschrieben isoliert. Mit Hilfe der Polymerasekettenreaktion wurde ein DNA Fragment mit einer Größe von 1200 bp, welches das rplK-Gen beinhaltet, amplifiziert. Dazu wurden auf Grundlage der SEQ ID No. 1 die folgenden Primer abgeleitet.
P1-up (Siehe auch SEQ ID No. 5): 5'- AGG AGC AGG CTG TTG TCA CC -3' P2-down: (Siehe auch SEQ ID No. 6):

5'- GCG GAT AGC TAC GTC GAT GG -3'

Die dargestellten Oligonukleotide wurden von der Firma ARK Scientific (ARK Scientific GmbH Biosystems, Darmstadt, Germany) synthetisiert und die PCR-Reaktion unter Verwendung der Pfu-DNA-Polymerase (Stratagene, La Jolla, USA) und des PTC 100-Thermocyclers (MJ Research Inc., Waltham, USA) durchgeführt.

Bei der PCR wurde ein Zyklus, bestehend aus thermischer Denaturierung (94°C, 90 Sekunden), Annealing (58°C, 90 Sekunden) und Polymerasereaktion (72°C, 90 Sekunden) 35 mal durchlaufen. Das so erhaltene 1200 bp DNA-Fragment wurde anschließend mit Hilfe des Qiagen PCR Purification Spin Kits (Qiagen, Hilden, Germany) aufgereinigt.

Für die Klonierung des rplK-Gens-enthaltenden DNAAmplifikats auf ein Plasmid, welches in C. glutamicum replizieren kann, wurde das Plasmid pECM3, ein Deletionsderivat des bei Tauch et al. (FEMS Microbiological Letters, 123, 343-347 (1994)) beschriebenen Plasmids pECM2, hergestellt. Dafür wurde das Plasmid pECM2 mit den Restriktionsenzymen BamHI (Amersham-Pharmacia, Freiburg, Germany) und BglII (Amersham-Pharmacia, Freiburg, Germany) restringiert und mit T4-Ligase (Amersham-Pharmacia, Freiburg, Germany), wie bei Sambrook et al. (Molecular Cloning: A Laboratory Manual, Cold Spring Habor Laboratory (1989)) beschrieben, behandelt, wodurch das Plasmid pECM3 erhalten wurde. Die Transformation des bei Grant et al. (Proceedings of the National Academy of Science USA, 87, 4645-4649 (1990)) beschriebenen E. coli-Stammes DH5αMCR mit dem Plasmid pECM3 erfolgte wie bei Tauch et al. (FEMS Microbiological Letters, 123, 343-347 (1994)) beschrieben. Die Transformanten wurden auf LBG-Agar (10 g Trypton, 5 g Hefe-Extrakt, 5 g NaCl, 2 g Glucose, 15 g Agar pro Liter), dem Chloramphenicol (Merck, Darmstadt, Germany) (50 mg/l) hinzugefügt worden war, selektioniert.

Anschließend wurde das rplK-Gen enthaltende 1200 bp DNA-Amplifikat mit dem Plasmid pECM3, welcher zuvor mit dem Restriktionsenzym SmaI (Amersham-Pharmacia, Freiburg, Germany) linearisiert wurde, gemischt und mit T4-DNA-Ligase (Amersham-Pharmacia, Freiburg, Germany) behandelt, wodurch das Plasmid prplK erhalten wurde. Die Transformation des E. coli-Stammes DH5αMCR mit dem Plasmid prplK erfolgte wie bei Tauch et al. (FEMS Microbiological Letters, 123, 343-347 (1994)) beschrieben, und die Transformanten wurden auf LBG-Agar, dem Chloramphenicol (Merck, Darmstadt, Germany) (50 mg/l) hinzugefügt worden war, selektioniert. Das Plasmid prplK trägt somit das vollständige rplK-Gen von C. glutamicum und kann sowohl in E. coli als auch in C. glutamicum autonom replizieren.

### Beispiel 4

### Einbau einer Deletion in das rplK-Gen

Mittels PCR-Technik wurde ein als ΔrplK bezeichnetes Allel des rplK-Gens hergestellt, das eine 12 bp lange Deletion trägt. Die erzeugte 12 bp Deletion im rplK-Gen führt zum Verlust des Tetrapeptids "Prolin-Alanin-Leucin-Glycin" im N-terminalen Bereich des L11-Proteins von C. glutamicum.

Als Primer für die Erzeugung des rplK-Deletionsalles wurden neben den in Beispiel 3 beschriebenen Primern P1-up und P2-down die im folgenden beschriebenen Oligonukleotide verwendet, die von der Firma ARK Scientific (ARK Scientific GmbH Biosystems, Darmstadt, Germany) hergestellt wurden.
P1-down (Siehe auch SEQ ID No. 7): 
5´-Extension-CGC CGT GAG C-5´-Seite-GCC AAC TGG AGG AGC AGG
GT-3 ´
 P2-up: (Siehe auch SEQ ID No. 8):

5´-Extension-TCC AGT TGG C-5´-Seite-GCT CAC GGC GTC AAC ATC
AG-3´

Die für die PCR verwendeten Primer wurden aus der bekannten DNA-Sequenz abgeleitet. Diese besitzen jeweils eine 10 bp 5'-Extension, die exakt komplementär zu den 5'-Seiten der Primer P1-up und P2-down ist. Aus C. glutamicum ATCC 13032 wurde die chromosomale DNA extrahiert und mit ihr als Matrix unter Verwendung der angegebenen Oligonukleotide P1-up , P1-down, P2-up und P2-down, der Pfu-DNA-Polymerase (Stratagene, La Jolla, USA), und des PTC 100-Thermocyclers (MJ Research Inc., Waltham, USA) zunächst in getrennten PCR-Reaktionen zwei 600 bp DNA-Fragmente erzeugt. Bei diesen PCR-Reaktionen wurde jeweils ein Zyklus, bestehend aus thermischer Denaturierung (94°C, 90 Sekunden), Annealing (58°C, 90 Sekunden) und Polymerasereaktion (72°C, 90 Sekunden) 35 mal durchlaufen.

Das erste 600 bp DNA-Amplifikat, als rplK-Teil 1 bezeichnet, wurde mit den Oligonukleotiden P1-up und P1 down erhalten. Es beinhaltet den 5'-Bereich des rplK-Gens (Nukleotid 1-87) und zusätzlich die vom Oligonukleotid P1-down stammende 10 bp-Extension, die den Nukleotiden 100-109 des rplK-Gens (SEQ ID No. 1) entspricht. Somit weist dieser amplizierte rplK-Genbereich eine 12 bp-Lücke im Vergleich zur verwendeten chromosomalen DNA-Template auf. Das zweite 600 bp DNA-Fragment, rplK-Teil 2, wurde mit den Oligonukleotiden P2-down und P2-up erhalten und beinhaltet die 3'-Region des rplK-Gens (Nukleotid 78-435) und trägt die identische Lücke (Nukleotid 88-99) innerhalb des amplifizierten rplK-Genbereichs. Diese beiden 600 bp DNA-Amplifikate weisen demzufolge einen 20 bp überlappenden DNA-Bereich auf. Die beiden 600 bp PCR-Produkte rplK-Teil 1 und rplK-Teil 2 wurden nun in einer weiteren PCR-Reaktion gemeinsam als DNA-Template verwendet, wobei sich aufgrund des überlappenden, komplementären DNA-Bereichs der "Hinstrang" von rplK-Teil 1 mit dem "Rückstrang" von rplK-Teil 2 verbinden konnte. Die Extension dieses überlappenden DNA-Bereichs bzw. die Zugabe der Oligonukleotide P1-up und P2-down, führte zur Erzeugung eines 1200 bp PCR-Amplifikats, welches ein 12 bp Deletionsderivat des C. glutamicum rplK-Gens beinhaltet. Bei diesen PCR-Reaktionen wurde jeweils ein Zyklus, bestehend aus thermischer Denaturierung (94°C, 90 Sekunden), Annealing (58°C, 90 Sekunden) und Polymerasereaktion (72°C, 90 Sekunden) 35 mal durchlaufen.

Die Nukleotidsequenz des ΔrplK-Allels ist in SEQ ID No. 3 und die von diesem Allel kodierte Variante des L11-Proteins in SEQ ID No. 4 dargestellt. Dieser L11-Proteinvariante fehlt das Tetrapeptid "Prolin-Alanin-Leucin-Glycin" entsprechend den Aminosäurepositionen 30 bis 33 der Wildtypform des L11-Proteins dargestellt in SEQ ID No. 2.

### Beispiel 5

### Einbau des ΔrplK-Allels in das Chromosom

Das ΔrplK-Allel, welches eine 12 bp Deletion im rplK-Gen beeinhaltet, wurde mittels Integrationsmutagenese unter Zuhilfenahme des bei Schäfer et al., Gene, 14, 69-73 (1994) beschriebenen sacB-Systems in das Chromosom von C. glutamicum eingebaut. Dieses System erlaubt dem Fachmann die Identifizierung bzw. Selektion von Allel-Austauschen, die sich durch homologe Rekombination vollziehen.

### 1. Konstruktion des Austauschvektors pΔrplK

Das in Beispiel 4 erhaltene 1200 bp rplK-Deletionsallel ΔrplK wurde mit Hilfe des Qiagen PCR Purification Spin Kits (Qiagen, Hilden, Germany) aufgereinigt und mit dem bei Schäfer et al., Gene, 14, 69-73 (1994) beschriebenen mobilisierbaren Kloniervektor pK18mobsacB zur Ligation eingesetzt. Dieser wurde zuvor mit dem Restriktionsenzym SmaI (Amersham-Pharmacia, Freiburg, Germany) linearisiert, mit dem rplK-Deletionsallel gemischt und und mit T4-DNA-Ligase (Amersham-Pharmacia, Freiburg, Germany) behandelt.

Es resultierte das Plasmid pΔrplK.

Die Transformation des E. coli-Stammes DH5α mit dem Plasmid pΔrplK erfolgte wie bei Tauch et al., FEMS Microbiological Letters, 123, 343-347 (1994) beschrieben. Die Transformanten wurden auf LBG-Agar, dem Kanamycin (Merck, Darmstadt, Germany) (50 mg/l) hinzugefügt worden war, selektioniert. Auf diese Weise entstand der Stamm DH5α/pΔrplK.

Ein Klon wurde ausgewählt und als ATCC13032ΔrplK bezeichnet.

### 2. Durchführung des Allel-Austauschs

Eine chromosomale 12 bp Deletion im rplK-Gen von C. glutamicum wurde mittels Integrationsmutagenese unter Zuhilfenahme des bei Schäfer et al., Gene, 14, 69-73 (1994) beschriebenen sacB-Systems erhalten. Dieses System erlaubt dem Fachmann die Identifizierung bzw. Selektion von Allel-Austauschen, die sich durch homologe Rekombination vollziehen.

Das mobilisierbare Plasmid pΔrplK wurde ausgehend von dem bei Simon et al., Bio/Technology, 1, 784-794 (1993) beschriebenen E. coli Donor-Stamm S17-1 mit Hilfe der von Schäfer et al., Journal of Bacteriology, 172, 1663-1666 (1990) beschriebenen Konjugationsmethode in den Stamm C. glutamicum ATCC 13032 als Rezipienten eingeführt. Da das Plasmid pΔrplK in C. glutamicum nicht replizieren kann, ist seine Etablierung nur durch Integration in das C. glutamicum-Chromosom via homologer Rekombination zwischen dem plasmidkodierten rplK-Deletionsfragment und dem identischen, chromosomalen rplK-Genbereich möglich. Die Transkonjuganten wurden auf LBG-Agar, dem Kanamycin (25 mg/l) (Merck, Darmstadt, Germany) und Nalidixinsäure (Merck, Darmstadt, Germany) (50 mg/l) hinzugefügt worden war, selektioniert.

Auf die anschließende Exzision des Plasmids pΔrplK mit Hilfe des sacB-Systems konnte nur unter Anwesenheit eines Wildtypallels von rplK selektioniert werden. Dazu wurde das in Beispiel 3 konstuierte Plasmid prplK, das das komplette rplK-Gen trägt, durch Elektroporation nach der Methode von Liebl et al. ,FEMS Microbiology Letters 65, 299-304 (1989) in den Integrantenstamm transferiert. Die Selektion des Stammes erfolgte auf LBG-Agar, dem Kanamycin (25 mg/l) (Merck, Darmstadt, Germany) und Chloramphenicol (10 mg/l) (Merck, Darmstadt, Germany) hinzugefügt worden war.

Eine ausgewählte, transformierte Kolonie wurde in 100 ml LBG-Flüssigmedium (in einem 250 ml-Erlenmeyerkolben mit Schikanen) überimpft und 24 Stunden bei 30°C und 300 Upm inkubiert. Anschließend wurden jeweils 2×10⁶ Zellen/ml dieser Flüssigkultur auf LBG-Agar, der 10% Sucrose (Merck, Darmstadt, Germany) enthielt, ausgebracht und 48 Stunden bei 30°C inkubiert. C. glutamicum-Zellen, die in der Lage waren auf diesem Medium zu wachsen, hatten das integrierte Plasmid pΔrplK in Folge eines zweiten Rekombinationsereignisses zwischen dem rplK-Deletionsallel und der natürlichen rplK-Region verloren. Dieses zweite Rekombinationsereignis führt entweder zur Wiederherstellung der natürlichen, chromosomalen rplK-Genanordnung, oder es resultiert in der Erzeugung einer C. glutamicum ΔrplK-Mutante, der das 12 bp N-terminale DNA-Fragment verlorengegangen ist. Von ausgewählten, "Sucrose-resistenten" und potentiellen pΔrplK-tragenden C. glutamicum-Zellen wurde die chromosomale DNA extrahiert. Diese diente als Matrix mit welcher unter Verwendung der rplK-Sequenz die Olidonukleotide Pdel-up und Pdel-down2 (ARK Scientific GmbH Biosystems, Darmstadt, Germany) abgeleitet wurden. Mit den Primern, der Pfu-DNA-Polymerase (Stratagene, La Jolla. USA) und des PTC 100-Thermocyclers (MJ Research Inc., Waltham, USA)wurden die PCR-Experimente durchgeführt. Bei der PCR wurde ein Zyklus, bestehend aus thermischer Denaturierung (94°C, 90 Sekunden), Annealing (58°C, 90 Sekunden) und Polymerasereaktion (72°C, 90 Sekunden) 35 mal durchlaufen, durchgeführt. Anschließend wurden die so erhaltenen DNA-Amplifikate mit Hilfe des Qiagen PCR Purification Spin Kits (Qiagen, Hilden, Germany) aufgereinigt. Die Analyse der Nukleotidsequenzen der aufgereinigten DNA-Amplifikate, die wie zuvor beschrieben durchgeführt wurde, erbrachte, daß in 43% der Fälle ein DNA-Amplifikat erzeugt worden war, dem der 12 bp DNA-Bereich fehlte. Demzufolge hatte in den zugehörigen pΔrplK-tragenden Transkonjuganten das zweite Rekombinationsereignis zur Erzeugung der chromosomalen 12 bp Deletion im rplK-Gen geführt.

Anschließend wurde aus einer ausgewählten, deletionstragenden Transkonjugante das Plasmid prplK durch die bei Schäfer et al., Journal of Bacteriology, 176, 7309-7319 (1994) beschriebene Methode des "Plasmid-Curings" entfernt. Der so erhaltene Stamm C. glutamicum ATCC13032 ΔrplK trägt somit eine chromosomale 12 bp Deletion innerhalb des rplK-Gens, was zu dem Verlust des Tetrapeptids "Prolin-Alanin-Leucin-Glycin" des L11-Proteins führt.

### Beispiel 6

### Herstellung von Lysin

Der in Beispiel 5 erhaltene Stamm C. glutamicum ATCC13032 ΔrplK wurde in einem zur Produktion von Lysin geeigneten Nährmedium kultiviert und der Lysingehalt im Kulturüberstand bestimmt.

Dazu wurde der Stamm zunächst auf Agarplatte (Hirn-Herz Agar) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII ( 10 g/l Bacto-Pepton, 10g /l Yeast Extract, 2,5 g/l NaCl, 20 g/l Glucose, pH 7,4) verwendet. Die Vorkultur wurde 24 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 OD betrug. Für die Hauptkultur wurde das Medium MM verwendet.

| Medium MM | |
|---|---|
| CSL (Corn Steep Liquor) | 5 g/l |
| MOPS (Morpholinopropansulfonsäure) | 20 g/l |
| Glucose (getrennt autoklaviert) | 50g/l |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄.* H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in einem 100 ml Erlenmeyerkolben mit Schikanen. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| ATCC13032 ΔrplK | 13,0 | 0,98 |
| ATCC13032 | 13,8 | 0,0 |

### Folgende Figuren sind beigefügt:

Figur 1: Karte des Plasmids pΔrplK.

Die verwendeten Abkürzungen und Bezeichnungen haben folgende Bedeutung.

| pdeltarplK | pΔrplK |
|---|---|
| sacB-Gen | sacB-Gen aus Bacillus suptilis, kodiert für das Enzym Levansucrase |
| | |
| lacZ-alpha : | Teil des 5'Ende des β-Galactosidase Gens |
| | |
| oriV | Replikationsursprung |
| | |
| KmR: | Kanamycin Resistenz |
| | |
| RP4 mob | mob-Region des Plasmids RP4 |
| | |
| BamHI: | Schnittstelle des Restriktionsenzyms BamHI |
| | |
| EcoRI: | Schnittstelle des Restriktionsenzyms EcoRI |
| | |
| ΔrplK | rplK-Allel mit einer Deletion von 12 bp im N-terminalen Bereich |

## Patentansprüche

1. Isoliertes Polynukleotid aus coryneformen Bakterien, enthaltend eine Polynukleotidsequenz, ausgewählt aus der Gruppe
a) Polynukleotid, das zu mindestens 70 % identisch ist mit einem Polynukleotid, das für ein Polypeptid kodiert, das die Aminosäuresequenz von SEQ ID No. 2 enthält,
b) Polynukleotid, das für ein Polypeptid kodiert, das eine Aminosäuresequenz enthält, die zu mindestens 70 % identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine replizierbare, bevorzugt rekombinante DNA ist.

3. Polynukleotid gemäß Anspruch 1, wobei
das Polynukleotid eine RNA ist.

4. Polynukleotid gemäß Anspruch 2,
enthaltend die Nukleinsäuresequenz wie in SEQ ID No. 1 dargestellt.

5. Polynukleotidsequenz gemäß Anspruch 2, die
für ein Polypeptid kodiert, das die Aminosäuresequenz wie in SEQ ID No. 2 dargestellt, enthält.

6. Polynukleotid gemäß Anspruch 1, insbesonder Punkt d, enthaltend die Nukleotidsequenz, wie in SEQ ID No. 3 dargestellt.

7. Polynukleotid gemäß Anspruch 1, insbesondere Punkt d, das für ein Polypeptid kodiert, das die in SEQ ID No. 4 dargestellte Aminosäuresequenz enthält.

8. Replizierbare DNA gemäß Anspruch 2,
enthaltend
(i) die Nukleotidsequenz, gezeigt in SEQ-ID-No. 1, oder
(ii) mindestens eine Sequenz, die der Sequenz (i) innerhalb des Bereichs der Degeneration des genetischen Codes einspricht, oder
(iii) mindestens eine Sequenz, die mit der zur Sequenz (i) oder (ii) komplementären Sequenz hybridisiert, und gegebenenfalls
(iv) funktionsgetreue Sinnmutanten in (i)

9. Vektor, enthaltend ein Polynukleotid gemäß Anspruch 1, hinterlegt in E.coli, DH5α/pΔrpk als DSM 13158, dargestellt in SEQ ID No. 3 und Figur 1.

10. Als Wirtszelle dienende coryneforme Bakterien, die eine Deletion oder eine Insertion in dem rplk-Gen enthalten.

11. Verfahren zur Herstellung von L-Aminosäuren, insbesondere L-Lysin,
**dadurch gekennzeichnet,**
**daß** man folgende Schritte durchführt,
a) Fermentation der die gewünschte L-Aminosäure produzierenden Bakterien, in denen man zumindest das rplk-Gen abschwächt,
b) Anreicherung des gewünschten L-Aminosäure im Medium
oder in den Zellen der Bakterien, und
c) Isolieren der L-Aminosäure.

12. Verfahren gemäß Anspruch 11,
**dadurch gekennzeichnet,**
**daß** man Bakterien einsetzt, in denen man zusätzlich weitere Gene des Biosyntheseweges der gewünschten L-Aminosäure verstärkt.

13. Verfahren gemäß Anspruch 11,
**dadurch gekennzeichnet,**
**daß** man Bakterien einsetzt, in denen die Stoffwechselwege zumindest teilweise ausgeschaltet sind, die die Bildung der gewünschten L-Aminosäure verringern.

14. Verfahren gemäß Anspruch 11,
**dadurch gekennzeichnet,**
**daß** man die Expression des Polynukleotids gemäß Anspruch 1, insbesondere 1 a bis 1 d verringert.

15. Verfahren gemäß Anspruch 11,
**dadurch gekennzeichnet,**
**daß** man die katalytischen Eigenschaften des Polypeptids (Enzymproteins) herabsetzt, für das das Polynukleotid gemäss Anspruch 1, insbesondere 1 a bis 1 d kodiert.

16. Verfahren gemäß Anspruch 11,
**dadurch gekennzeichnet,**
**daß** man Bakterien einsetzt, in denen man zur Abschwächung eine Insertionsmutagenese unter Verwendung des Plasmids pΔrpk, dargestellt in Figur 1 und hinterlegt als DSM 13158 erzeugt.

17. Verfahren gemäß Anspruch 11,
**dadurch gekennzeichnet,**
**daß** man für die Herstellung von L-Aminosäuren, insbesondere L-Lysin, Bakterien fermentiert, in denen man gleichzeitig eines oder mehrere der Gene überexprimiert, ausgewählt aus der Gruppe
17.1 das für die Dihydrodipicolinat-Synthase kodierende dapA-Gen,
17.2 ein für eine feed back resistente Aspartatkinase,
17.3 das die S-(2-Aminoethyl)-Cystein-Resistenz vermittelnde DNA-Fragment,
17.4 das die Pyruvat-Carboxylase kodierende pyc-Gen,
17.5 pyc-Gen (Eikmanns (1992) Journal of Bacteriology)
17.6 das für die Malat:Chinon Oxidoreduktase kodierende mqo-Gen
17.7 das für den Lysin-Export kodierende lysE-Gen,
17.8 das zwa1-Gen.

18. Verfahren gemäß Anspruch 11,
**dadurch gekennzeichnet,**
**daß** man für die Herstellung von Aminosäuren, insbesondere von L-Lysin, Bakterien fermentiert, in denen man gleichzeitig eines oder mehrere der Gene, ausgewählt aus der Gruppe
18.1 das für die Phosphoenolpyruvat-Carboxykinase kodierende pck-Gen,
18.2 das für die Glucose-6-Phosphat Isomerase kodierende pgi-Gen,
18.3 das für die Pyruvat-Oxidase kodierende poxB-Gen,
18.4 das zwa2-Gen oder
18.5 das für die PPGPP-Synthetase I kodierende rela-Gen
abschwächt.

19. Verfahren gemäß einem oder mehreren der vorhergehenden Ansprüche,
**dadurch gekennzeichnet,**
**daß** man Mikroorganismen der Gattung Corynebacterium glutamicum einsetzt.

20. Verwendung von Polynukleotidsequenzen gemäß Anspruch 1 als Primer zur Herstellung der DNA von Genen, die eine dem rplK-Gen entsprechende Wirkung entfalten, durch die Polymerase-Kettenreaktion.

21. Verwendung von Polynukleotidsequenzen gemäß Anspruch 1 als Hybridisierungssonden.


SEQUENZPROTOKOLL

<110> Degussa-Hüls AG

<120> Neue für das rplK-Gen kodierende Nukleotidsequenzen

<130> 990178 BT

<140>
<141>

<160> 8

<170> PatentIn Ver. 2.1

<210> 1
<211> 835
<212> DNA
<213> Corynebacterium glutamicum

<220>
<221> CDS
<222> (201)..(635)
<223> rplK-Gen

<400> 1
ttgcgtgtag ggtagacaat cgcgtgtttt ttaagcatgc tcaaaatcat tcatccccgg 60

tggcccggtt acgtaaagat cagcaaagat gatcaactaa agcgatcatc tgaagttgta 120

gcgggaccga gcatccggac ggttactagt ggggtttcat cgtcccagtt gtggccggta 180

acaaggaagc aggtttaacg atg gct cct aag aag aag aag aag gtc act ggc 233
Met Ala Pro Lys Lys Lys Lys Lys Val Thr Gly
1               5                   10

ctc atc aag ctc cag atc cag gca gga cag gca aac cct gct cct cca 281
Leu Ile Lys Leu Gln Ile Gln Ala Gly Gln Ala Asn Pro Ala Pro Pro
            15                  20                  25

gtt ggc cca gca ctt ggt gct cac ggc gtc aac atc atg gaa ttc tgc 329
Val Gly Pro Ala Leu Gly Ala His Gly Val Asn Ile Met Glu Phe Cys
        30                  35                  40

aag gct tac aac gct gcg act gaa aac cag cgc ggc aac gtt gtt cct 377
Lys Ala Tyr Asn Ala Ala Thr Glu Asn Gln Arg Gly Asn Val Val Pro
    45                  50                  55

gtt gag atc acc gtt tac gaa gac cgt tca ttc gac ttc aag ctg aag 425
Val Glu Ile Thr Val Tyr Glu Asp Arg Ser Phe Asp Phe Lys Leu Lys
60                  65                  70                  75

act cct cca gct gca aag ctt ctt ctg aag gct gct ggc ctg cag aag 473
Thr Pro Pro Ala Ala Lys Leu Leu Leu Lys Ala Ala Gly Leu Gln Lys
                80                  85                  90

ggc tcc ggc gtt cct cac acc cag aag gtc ggc aag gtt tcc atg gct 521
Gly Ser Gly Val Pro His Thr Gln Lys Val Gly Lys Val Ser Met Ala
            95                  100                 105

cag gtt cgt gag atc gct gag acc aag aag gaa gac ctg aac gct cgc 569
Gln Val Arg Glu Ile Ala Glu Thr Lys Lys Glu Asp Leu Asn Ala Arg
        110                 115                 120

gat atc gac gct gct gcg aag atc atc gct ggt acc gct cgt tcc atg   617
Asp Ile Asp Ala Ala Ala Lys Ile Ile Ala Gly Thr Ala Arg Ser Met
    125                 130                 135

ggc atc acc gtc gaa ggc taaaagcttt cacaccggtt agtggctcat          665
Gly Ile Thr Val Glu Gly
140                 145

tcaaaatgaa tggccaccaa ccaattttca ccaaagtttt atgtggcagg gccagctccg 725

gcccgttaaa ccacagaatt ccatgaaagg gaatttctaa tgagcaagaa ctctaaggcg 785

taccgcgagg ccgctgagaa gatcgacgct ggtcgcatct actccccact            835

<210> 2
<211> 145
<212> PRT
<213> Corynebacterium glutamicum

<400> 2
Met Ala Pro Lys Lys Lys Lys Lys Val Thr Gly Leu Ile Lys Leu Gln
  1               5                  10                  15

Ile Gln Ala Gly Gln Ala Asn Pro Ala Pro Pro Val Gly Pro Ala Leu
             20                  25                  30

Gly Ala His Gly Val Asn Ile Met Glu Phe Cys Lys Ala Tyr Asn Ala
         35                  40                  45

Ala Thr Glu Asn Gln Arg Gly Asn Val Val Pro Val Glu Ile Thr Val
     50                  55                  60

Tyr Glu Asp Arg Ser Phe Asp Phe Lys Leu Lys Thr Pro Pro Ala Ala
 65                  70                  75                  80

Lys Leu Leu Leu Lys Ala Ala Gly Leu Gln Lys Gly Ser Gly Val Pro
                 85                  90                  95

His Thr Gln Lys Val Gly Lys Val Ser Met Ala Gln Val Arg Glu Ile
            100                 105                 110

Ala Glu Thr Lys Lys Glu Asp Leu Asn Ala Arg Asp Ile Asp Ala Ala
        115                 120                 125

Ala Lys Ile Ile Ala Gly Thr Ala Arg Ser Met Gly Ile Thr Val Glu
    130                 135                 140

Gly
145

<210> 3
<211> 825
<212> DNA
<213> Corynebacterium glutamicum

<220>
<221> CDS
<222> (200)..(622)
<223> delta rplK

<400> 3
tgcgtgtagg gtagacaatc gcgtgttttt taagcatgct caaaatcatt catccccggt 60

ggcccggtta cgtaaagatc agcaaagatg atcaactaaa gcgatcatct gaagttgtag 120

cgggaccgag catccggacg gttactagtg gggtttcatc gtcccagttg tggccggtaa 180

caaggaagca ggtttaacg atg gct cct aag aag aag aag aag gtc act ggc 232
Met Ala Pro Lys Lys Lys Lys Lys Val Thr Gly
1               5                   10

ctc atc aag ctc cag atc cag gca gga cag gca aac cct gct cct cca 280
Leu Ile Lys Leu Gln Ile Gln Ala Gly Gln Ala Asn Pro Ala Pro Pro
            15                  20                  25

gtt ggc gct cac ggc gtc aac atc atg gaa ttc tgc aag gct tac aac 328
Val Gly Ala His Gly Val Asn Ile Met Glu Phe Cys Lys Ala Tyr Asn
        30                  35                  40

gct gcg act gaa aac cag cgc ggc aac gtt gtt cct gtt gag atc acc 376
Ala Ala Thr Glu Asn Gln Arg Gly Asn Val Val Pro Val Glu Ile Thr
    45                  50                  55

gtt tac gaa gac cgt tca ttc gac ttc aag ctg aag act cct cca gct 424
Val Tyr Glu Asp Arg Ser Phe Asp Phe Lys Leu Lys Thr Pro Pro Ala
60                  65                  70                  75

gca aag ctt ctt ctg aag gct gct ggc ctg cag aag ggc tcc ggc gtt 472
Ala Lys Leu Leu Leu Lys Ala Ala Gly Leu Gln Lys Gly Ser Gly Val
                80                  85                  90

cct cac acc cag aag gtc ggc aag gtt tcc atg gct cag gtt cgt gag 520
Pro His Thr Gln Lys Val Gly Lys Val Ser Met Ala Gln Val Arg Glu
            95                  100                 105

atc gct gag acc aag aag gaa gac ctg aac gct cgc gat atc gac gct 568
Ile Ala Glu Thr Lys Lys Glu Asp Leu Asn Ala Arg Asp Ile Asp Ala
        110                 115                 120

gct gcg aag atc atc gct ggt acc gct cgt tcc atg ggc atc acc gtc 616
Ala Ala Lys Ile Ile Ala Gly Thr Ala Arg Ser Met Gly Ile Thr Val
    125                 130                 135

gaa ggc taaaagcttt cacaccggtt agtggctcat tcaaaatgaa tggccaccaa 672
Glu Gly
140

ccaattttca ccaaagtttt atgtggcagg gccagctccg gcccgttaaa ccacagaatt 732

ccatgaaagg gaatttctaa tgagcaagaa ctctaaggcg taccgcgagg ccgctgagaa 792

gatcgacgct ggtcgcatct actccccact cga 825


<210> 4
<211> 141
<212> PRT
<213> Corynebacterium glutamicum

<400> 4
Met Ala Pro Lys Lys Lys Lys Lys Val Thr Gly Leu Ile Lys Leu Gln
1               5                   10                  15

Ile Gln Ala Gly Gln Ala Asn Pro Ala Pro Pro Val Gly Ala His Gly
            20                  25                  30

Val Asn Ile Met Glu Phe Cys Lys Ala Tyr Asn Ala Ala Thr Glu Asn
        35                  40                  45

Gln Arg Gly Asn Val Val Pro Val Glu Ile Thr Val Tyr Glu Asp Arg
    50                  55                  60

Ser Phe Asp Phe Lys Leu Lys Thr Pro Pro Ala Ala Lys Leu Leu Leu
65                  70                  75                  80

Lys Ala Ala Gly Leu Gln Lys Gly Ser Gly Val Pro His Thr Gln Lys
                85                  90                  95

Val Gly Lys Val Ser Met Ala Gln Val Arg Glu Ile Ala Glu Thr Lys
            100                 105                 110

Lys Glu Asp Leu Asn Ala Arg Asp Ile Asp Ala Ala Ala Lys Ile Ile
        115                 120                 125

Ala Gly Thr Ala Arg Ser Met Gly Ile Thr Val Glu Gly
    130                 135                 140

<210> 5
<211> 20
<212> DNA
<213> Künstliche Sequenz

<220>
<223> Primer P1-up

<220>
<223> Beschreibung der künstlichen Sequenz: Primer

<400> 5
aggagcaggc tgttgtcacc 20

<210> 6
<211> 20
<212> DNA
<213> Künstliche Sequenz

<220>
<223> P2-down

<220>
<223> Beschreibung der künstlichen Sequenz: Primer

<400> 6
gcggatagct acgtcgatgg 20

<210> 7
<211> 30
<212> DNA
<213> Künstliche Sequenz

<220>
<223> P1-down

<220>
<223> Beschreibung der künstlichen Sequenz: Primer

<400> 7
cgccgtgagc gccaactgga ggagcagggt 30

<210> 8

<211> 30
<212> DNA
<213> Künstliche Sequenz

<220>
<223> P2-up

<220>
<223> Beschreibung der künstlichen Sequenz: Primer

<400> 8
tccagttggc gctcacggcg tcaacatcag 30